# FASCICULE DE BREVET EUROPEEN

(11) **EP 0 948 340 B1**
(45) Date de publication et mention de la délivrance du brevet: **20.03.2002**
(21) Numéro de dépôt: 97952968.2
(22) Date de dépôt: 22.12.1997
(51) Int. Cl.: A61K 35/78

(54) **SUBSTANCES A ACTIVITE BIOLOGIQUE, LEUR PROCEDE D'OBTENTION ET LES COMPOSITIONS EN RENFERMANT**
BIOLOGISCH AKTIVE SUBSTANZEN, VERFAHREN ZUR HERSTELLUNG UND SIE ENTHALTENDE ZUSAMMENSETZUNGEN
BIOLOGICALLY ACTIVE SUBSTANCES, METHOD FOR OBTAINING AND COMPOSITIONS CONTAINING THEM

(30) Priorité: 27.12.1996 FR 9616116
(43) Date de publication de la demande: 13.10.1999
(73) Titulaire: Inovat Sarl, Tunis 1002 (TN)
(72) Inventeur: GUTIERREZ, Gilles, F-69006 Lyon (FR); SERRAR, Mostafa, F-69100 Villeurbanne (FR); VIORNERY, Lionel, F-69006 Lyon (FR); NORTON, James, Worschire CV7 8AH (GB)
(74) Mandataire: Burtin, Jean-François
(86) Numéro de dépôt international: FR9702375
(87) Numéro de publication internationale: WO9829128

(56) Documents cités:
- DE-A- 1 617 409

## Description

La présente invention se rapporte au domaine de la biochimie, et plus précisément à des composés biologiques d'origine végétale.

La présente invention concerne de nouvelles substances à activité biologique extraites de la peau de fruits des plantes à métabolisme CAM, leur procédé d'obtention et les compositions pharmaceutiques, cosmétiques et/ou alimentaires en renfermant.

Les compositions de la présente invention sont destinées à accélérer et amplifier la synthèse des protéines de choc thermique (ou protéines de stress) des cellules des êtres vivants. En effet, les conditions de stress ou d'agression sont de nature à altérer la qualité de la synthèse des protéines. Les eucaryotes ont été amenés à développer des voies métaboliques destinées à préserver leur structure.

Les plantes à métabolisme CAM appartiennent principalement aux familles des Cactacées, des Crassulacées et des Saxifragacées, qui forment la seule entité susceptible de développer ce type de métabolisme.
Ces plantes sont particulièrement adaptées aux conditions climatiques sévères et possèdent un métabolisme chlorophyllien particulier faisant intervenir un cycle du carbone, appelé CAM (« Crassulacees Acid Metabolism »).

Certaines plantes sont aptes à vivre dans des conditions stressantes (c'est notamment le cas des plantes vivant dans les zones arides des déserts chauds ou froids ou dans les zones d'altitude), et possèdent un métabolisme particulier d'incorporation du CO₂, appelé CAM. Les plantes de la famille des Cactacées, des Crassulacées et des Saxifragacées sont bien adaptées à ces conditions de stress thermique. La synthèse des substances biologiques activant ou amplifiant l'expression des protéines de choc thermique est surtout abondante dans le fruit lors de sa formation ; la maturation du fruit se fait surtout en période de retour à une température normale. Le fruit étant très exposé à la chaleur, il y a tout lieu de penser qu'il dispose de moyens pour protéger ses synthèses protéiques.

Cette mesure de protection pourrait se retrouver également dans d'autres espèces vivantes.
Parmi les plantes à métabolisme CAM, on citera particulièrement les Cactacées. Les Cactacées constituent une famille de plantes dicotylédones, communément appelées cactus, appréciées pour leurs formes curieuses et leurs fleurs brillamment colorées. Un des genres les plus répandus de la famille des Cactacées est constitué par les Opuntias. L'Opuntia ficus-indica ou figuier de Barbarie (famille des Cactacées) est formé d'articles aplatis, de forme ovale, les « raquettes », dont la surface est parsemée d'aréoles. Chacune d'elles comprend un point végétatif dormant, protégé par des soies, des poils barbelés ou glochidies (caractéristiques du genre), des épines ; elles constituent ainsi l'homologue des bourgeons. Sur les jeunes pousses, les aréoles sont axillées par de petites feuilles charnues, rapidement caduques.

La croissance et la prolifération des organismes vivants supposent une grande autonomie non seulement pour restaurer le matériel génétique, garant de la pérennité de l'espèce, mais aussi pour réparer les lésions de leur corps, afin de préserver leur intégrité. Ces deux nécessités font appel à des systèmes biologiques complémentaires.

On a déjà découvert divers mécanismes utilisés par les cellules pour assurer la conservation intacte du patrimoine génétique ; à titre d'exemple, on peut citer le système de la protéine p 53. De même, il existe des mécanismes pour assurer la conformité structurale des protéines élaborées à partir de la transcription des informations contenues dans le génome. Ces mécanismes de protection des synthèses utilisent divers procédés et, parmi ceux-ci, la synthèse de ce que l'on appelle « protéines de choc thermique », malgré qu'elles ne répondent pas uniquement de manière spécifique aux stress thermiques.
Le terme de stress doit être pris au sens large : il comprend des stress de diverses origines, qu'il s'agisse d'élévation de température (d'où le nom de protéines de choc thermique), de stress oxydatifs, d'irradiations aux UV, d'infections virales ou bactériennes, ou de stress consécutifs à divers stimuli tels qu'une déprivation nutritionnelle, une agression mécanique ou un choc métabolique. C'est pourquoi, les protéines de choc thermique sont synthétisées par les cellules pour répondre aussi bien aux agressions d'origine chimique (poisons, ...), physique (rayonnements ionisants, osmolarité, pH, ...), biologique (cytokines), ou mécanique.

Les protéines de choc thermique (HSP) sont des protéines chargées de protéger la cellule, et plus particulièrement ses synthèses protéiques, des agressions de toutes origines.
Avec l'âge, les capacités de synthèse protéique diminuent considérablement, et de ce fait l'aptitude à répondre à une agression est de moins en moins adaptée. Ceci a pour conséquence la mort de nombreuses cellules, le défaut de protection, et une diminution du capital cellulaire, qui se traduit, notamment au niveau de la peau des mammifères, par la diminution du nombre de kératinocytes disponibles pour protéger le derme. Le temps, l'exposition au soleil et les variations de température induisent des agressions agressant les cellules. La peau du jeune adulte humain est particulièrement bien adaptée pour répondre rapidement à l'agression car elle est capable de synthétiser de grandes quantités de protéines de choc thermique pour protéger la conformation des protéines au cours de la synthèse. Avec l'âge, les capacités de synthèse des protéines de choc thermique diminuent.
La protection solaire de la peau est l'un des soins les plus demandés par les consommateurs et les plus recommandés par les prescripteurs. La variabilité de la couche d'ozone protectrice, la recherche de la chaleur estivale, les possibilités de voyage sous les tropiques ont considérablement aggravé le risque d'apparition des troubles cutanés ayant pour origine l'exposition solaire et l'agression thermique. Aujourd'hui, la protection vis-à-vis des rayonnements UV et IR existe sous différentes possibilités de filtration ou de réflexion, mais, il n'existe pas de produits à appliquer sur la peau, pour la protéger contre la chaleur ou un froid excessif.

Le corps utilise les protéines de choc thermique pour prévenir les dommages induits par de nombreux types d'agression : agressions venant de l'extérieur (rayonnement photolumineux, thermique, composés chimiques ou biologiques), mais aussi agressions venant de l'intérieur (hyperthermie, hypothermie, variation des paramètres biologiques, comme par exemple l'osmolarité, la concentration en certaines substances, etc.).

Les protéines de choc thermique sont aussi appelées protéines de stress, protéines de choc ou, en terminologie anglo-saxonne, « heat shock protein » ; la terminologie scientifique les dénomme HSP. Ces protéines peuvent encore être appelées, pour certaines, protéines chaperonnes, et pour d'autres, « glucose regulated protein » (GRP).

Les protéines de choc thermique (HSP) regroupent un très grand nombre de molécules. Aussi, pour simplifier leur dénomination, il est convenu de les définir spécifiquement par leur poids moléculaire, exprimé en kilodaltons (kDa). On distingue ainsi différents groupes de HSP : les hsp 110, (110 kDa), hsp 90, (90 kDa), hsp 70, (70 kDa) qui représentent la plus grande famille de hsp ; les hsp 60, (60 kDa), hsp 47, (47 kDa) et les hsp de petite taille (soit entre 16 et 40 kDa).
Les protéines de choc thermique constituent une grande famille moléculaire intervenant dans le maintien de la conformation spatiale des protéines. C'est ainsi que lors d'une élévation de température, les protéines voient leur structure spatiale modifiée (il s'agit du même phénomène que la coagulation de l'albumine en milieu acide).
Le grand nombre de HSP provient du fait qu'elles agissent avec une spécificité qui dépend du type cellulaire, de leur destination ou encore du type de stress. Ainsi, chaque membre de la famille des HSP a une fonction bien précise et les points d'intervention sont très nombreux. Les HSP s'appliquent non seulement aux étapes post-traductionnelles s'inscrivant dans une fonction normale de la synthèse et de la vie des protéines, comme la mise en place de la conformation et leur transport, mais aussi au niveau d'événements altérant la synthèse protéique en intervenant dans la sélection des voies métaboliques : restauration, destruction et élimination des substrats modifiés. Certains HSP ont une action qui se développe lors d'une agression thermique, d'autres ne jouent leur rôle qu'en présence d'un risque chimique.
Il existe donc un grand nombre de protéines de choc, chacune adaptée à un mécanisme particulier de protection.

Ces substances, de nature protéique, sont codées par des gènes pour lesquels la localisation chromosomique n'est établie que pour quelques uns. Il faut noter que ces substances sont maintenues en l'état d'une manière tout à fait constante tout au long de la phylogénie tant sur le plan de la séquence que de la fonction, ce qui veut dire que les conséquences de la stimulation ou les modifications de la synthèse se retrouvent non seulement avec des cellules humaines, mais sans doute avec les cellules de toutes les espèces animales.

Bien que les HSP 70 soient synthétisées de manière constitutive, pour résister aux agressions, les cellules synthétisent, en cas de nécessité, de grandes quantités de ces protéines. Il est donc possible de traiter des cellules par ces substances protéiques pour améliorer leur viabilité face à un stress. On peut donc intervenir sur l'amélioration de la viabilité cellulaire en ajoutant ces substances soit dans le milieu nutritif, soit au coeur même des cellules. Leur action a été étudiée, grâce à l'ingénierie génétique ou la biologie moléculaire, en vectorisant les gènes codant pour les HSP.

Les techniques d'addition présentent de nombreux inconvénients pour les organismes pluricellulaires:
- il n'est pas possible de cibler les cellules concernées,
- la substance peut être détruite dans les milieux extracellulaires,
- il n'est pas possible d'apporter une protéine dans de nombreuses cellules.
Les techniques de biologie moléculaire présentent elles aussi des inconvénients majeurs :
- le très grand nombre d'HSP et leur spécificité rendent les opérations délicates, nombreuses et fastidieuses,
- l'amplification du génome-codant court-circuite les régulations,
- il n'est pas possible aujourd'hui de contrôler la synthèse de la protéine,
- lors de la transfection génique, la protéine est toujours surexprimée quelles que soient les circonstances.

La production des HSP par induction de la transcription (synthèse des ARNm à partir de l'ADN) ou de la traduction (synthèse des protéines par les ribosomes à partir des ARNm) est possible si l'on dispose de substances pharmacologiques qui agissent soit au niveau de la membrane plasmique, soit dans le cytoplasme, soit encore au niveau nucléaire.

La Demanderesse a trouvé, dans les plantes à métabolisme CAM, et plus particulièrement dans le fruit des plantes de la famille des Cactacées, des substances susceptibles de protéger les synthèses protéiques. Après diverses recherches, il a été découvert, d'une manière surprenante, que ces substances étaient reconnues par les cellules animales.

La présente invention a pour objet de nouvelles substances à activité biologique, notamment sur le métabolisme des HSP, non cyto-toxiques, isolées des plantes à métabolisme CAM, comme les plantes de la famille des Cactacées, des Crassulacées et des Saxifragacées.
La présente invention concerne notamment les substances obtenues de la peau de fruits des plantes de la famille des Cactacées, et plus précisément de la peau de fruits d'Opuntia, et notamment celle de la figue de Barbarie.
La présente invention concerne encore un procédé d'obtention des substances à activité biologique, non-cytotoxiques, isolées des plantes à métabolisme CAM.
Le procédé d'obtention des substances à activité biologique, non cyto-toxiques, est caractérisé en ce que l'on soumet un fruit d'une plante à métabolisme CAM à un séchage, puis à une déshydratation, suivie d'un épuisement par un solvant organique polaire pour obtenir un extrait organique de fruit, dilue cet extrait avec de l'eau, purifie l'extrait dilué par épuisement à l'hexane, sépare la fraction organique hexanique que l'on évapore à sec sous vide, et obtient la fraction active du fruit de plante à métabolisme CAM, à savoir celle contenant lesdites substances à activité biologique. La fraction active est encore séchée, et éventuellement reprise par un solvant biocompatible tel que l'éthanol, ou par un solvant approprié selon les opérations souhaitées.

On utilise le fruit de la plante à métabolisme CAM et notamment la peau du fruit des plantes de la famille des Cactacées. Le fruit de la plante à métabolisme CAM est un fruit de la plante de la famille des Cactacées, et notamment le fruit d'Opuntia, et spécifiquement la figue de Barbarie.

Il ressort du document DE 1617409 que les cladodes d'Opuntia indica, c'est-à-dire des rameaux aplatis simulant des feuilles, ont déjà été utilisées en thérapeutique, en particulier comme agent anti-viral non citotoxique et administré par voie topique.
Cependant, rien dans la littérature antérieure ne permettait de déduire que les fruits, et plus particulièrement les peaux de fruits d'Opuntia, renfermaient un principe actif qui intervient dans la synthèse des HSP.
On ne trouve pas davantage d'information dans la littérature sur le fait que les substances extraites de la peau de fruit d'Opuntia indica sont susceptibles d'accélérer et d'amplifier la synthèse des HSP dans des milieux de culture cellulaire ou dans les cellules des êtres vivants *in vivo* ou *in vitro.*

Le procédé d'obtention de la ou des substances actives se caractérise encore par le fait que l'on peut utiliser la peau du fruit mature ou non mature ; la peau du fruit est obtenue par pelage, érosion ou broyage du fruit.
Le solvant organique polaire mis en oeuvre dans le procédé d'obtention est de préférence un solvant choisi parmi les alcools aliphatiques et les cétones aliphatiques miscibles à l'eau comme l'éthanol et/ou l'acétone. L'extraction directe de la plante par un solvant peu ou non polaire peut être aussi une alternative qui comporte un affrontement avec un solvant polaire.

Les substances à activité biologique selon l'invention, non cyto-toxiques, extraites de plantes à métabolisme CAM comme les plantes de la famille des Cactacées, des Crassulacées et des Saxifragacées, et notamment celles extraites de la peau de fruits d'Opuntia, et spécifiquement de celle de figue de Barbarie, manifestent des propriétés pharmacologiques très intéressantes, notamment sur le métabolisme des HSP, et trouvent de ce fait un emploi en thérapeutique, en cosmétique et/ou en alimentation.
Ces substances non cyto-toxiques, extraites de plantes à métabolisme CAM comme les Cactacées, les Crassulacées, les Saxifragacées agissent sur les cellules des tissus ou des êtres vivants en accélérant et/ou en amplifiant la synthèse des protéines de choc thermique (HSP), lorsque la cellule est soumise à un stress ou à diverses agressions mettant en jeu la synthèse des protéines de choc thermique.
Il est important d'insister sur la non cyto-toxicité de ces substances, car la plupart des substances cyto-toxiques induisent une augmentation de la synthèse des protéines de choc thermique (HSP).
Les substances de la présente invention sont destinées à traiter les cellules des tissus ou des êtres vivants, pour améliorer leur résistance à un stress ou à diverses agressions qui induisent la synthèse des protéines de choc thermique, ou pour prévenir et/ou traiter les conséquences d'un stress ou de diverses agressions qui induisent la synthèse des protéines de choc thermique et qui modifient la synthèse des protéines.

D'une manière générale, les substances de la présente invention sont destinées à prévenir et/ou à traiter toute conséquence d'une manifestation entraînant un stress ou une agression cellulaire.
Ces substances trouvent un emploi dans un domaine très large de la thérapeutique, de la cosmétique et/ou de l'alimentation.

Ainsi, les substances de la présente invention sont utilisables dans la prévention et/ou le traitement des conséquences d'ischémies vasculaires ; de fibroses cutanées (chéloïdes), pulmonaires, hépatiques ; d'interventions chirurgicales (effets secondaires dus aux traitements par chimiothérapie, aux rayonnements ionisants ou à la radiothérapie, etc.), de la ménopause ; des agressions par les radicaux libres ; des lésions mécaniques : étirement d'un muscle, de la peau (vergetures) ; des frottements mécaniques (chevaux, attelage, chaussures, vêtements, etc.) ; des rayonnements thermiques, photoniques; la prévention des risques liés à l'activité thérapeutique, à la complication de certaines maladies (hépatites virales, cancers, infections diverses, maladies dégénératives impliquant un phénomène apoptotique...), à certaines activités humaines professionnelles comme celles des pompiers ou des travailleurs susceptibles de subir des rayonnements ionisants, comme la plongée subaquatique et autres, etc.
A ces fins, une ou plusieurs substances selon l'invention, isolées de la peau de fruits des plantes à métabolisme CAM comme les plantes de la famille des Cactacées, des Crassulacées, et des Saxifragacées, et notamment de la peau de fruits d'Opuntia, et spécifiquement de la figue de Barbarie, sont utilisées seules ou en association ou en mélange avec un ou plusieurs principes actifs à action complémentaire ainsi qu'avec un excipient ou un véhicule inerte, non toxique, approprié à l'usage envisagé, en vue de la réalisation de compositions pharmaceutiques, cosmétiques et/ou alimentaires.

La présente invention concerne encore l'utilisation de la ou des substances à activité biologique, pour accélérer et amplifier la synthèse des protéines de choc thermique (HSP) dans des milieux de culture cellulaires.

Les compositions selon l'invention sont destinées à l'administration par voie digestive, parentérale, topique ou rectale. Les compositions sont donc présentées sous forme de comprimés nus ou enrobés, de dragées, de capsules, de gélules, de pilules, de cachets, de sirops, de poudres à ingérer ou à usage externe, de compositions adjuvantes pour la cicatrisation postopératoire, pour les brûlures, les traumatismes ; de suppositoires ; de solutés ou de suspensions injectables conditionnés en ampoules ; de crèmes, de gels ou de pommade.

Les principes actifs complémentaires qui conviennent pour de telles administrations et pour de telles compositions sont par exemple les filtres solaires, les antiseptiques, les antibiotiques, les analgésiques, etc.
Les excipients qui conviennent pour de telles administrations et pour de telles compositions sont des adjuvants de fabrication (liants, fluidifiants...), des agents de sapidité, des colorants et des stabilisants, etc.

Pour l'usage thérapeutique, cosmétique et/ou alimentaire, la teneur en principe actif (à savoir les substances à activité biologique selon l'invention) s'échelonne, en équivalent de plantes sèches, de 10 x 10⁻³ mg/ml à 400 mg/ml, en particulier de 0,1 mg/ml à 200 mg/ml, et de préférence de 0,5 mg/ml à 100 mg/ml par prise unitaire.
La posologie journalière s'échelonne de 0,1 mg/kg à 200 mg/kg par jour, en fonction de l'indication thérapeutique, cosmétique et/ou alimentaire, et de la voie d'administration.

La plante et l'extrait sec n'étant pas toxiques, la limite supérieure de la dose administrée est limitée seulement par les capacités d'ingestion, la solubilité, la disponibilité biologique et pharmacologique ou encore par l'effet associé à au moins un autre principe actif présent dans la plante et indépendant de la fraction active.
Les exemples suivants illustrent l'invention. Ces exemples sont décrits en tenant compte des figures annexées.

### EXEMPLE 1

### Amplification de la synthèse d'HSP 90 des fibroblastes soumis à un traitement thermique.

Les protéines de choc thermique (HSP) sont indispensables à la préservation de la synthèse des protéines lors d'une agression extérieure. L'augmentation de la synthèse des HSP permet de mieux protéger la cellule, par exemple au moment d'une augmentation de température.

Dans le cadre de l'expérience décrite ci-après, on a recherché des substances susceptibles d'amplifier la synthèse de l'HSP 90 lors d'une élévation de la température à 43°C sans induire la synthèse de cette molécule à 37°C (condition normale) pour éliminer l'induction d'éventuelles productions de HSP par une substance toxique.

La substance étudiée ci-dessous provient d'un extrait alcoolique obtenu à partir de la peau de fruits d'Opuntia ficus-indica ou figue de Barbarie. Cette substance est dénommée ci-après « OE », ce qui signifie « Opuntia Extract » ou extrait d'Opuntia.

Pour ce faire, on a traité 100 g de plante déshydratée ou de fruit entier déshydraté ou encore 1g de peau de fruit déshydraté, avec 10 fois son poids en solvant organique polaire. On ajoute 10 µl de l'extrait obtenu à 1 ml de milieu approprié à la culture des kératinocytes ou des fibroblastes. Après incubation d'une nuit à 37 °C, la synthèse des HSP n'est pas modifiée, mais après une exposition à une température de 43 °C, les HSP apparaissent de manière beaucoup plus précoce.
A titre d'exemple, HSP 90 révélé par immunofluorescence indirecte apparaît entre 10 et 20 mn dans une culture de fibroblastes incubés en présence d'extrait de Cactacées (O.E) alors que les milieux de culture contenant des cellules non incubées ne montrent HSP 90 qu'une à deux heures plus tard.
Les valeurs des tableaux et des graphes 1, 2, 3 et 4 sont exprimées en unités arbitraires en fonction de la surface de fluorescence pour un seuil fixé pour l'ensemble des observations. Il s'agit de valeurs relatives en unités arbitraires établies à partir d'un seuil de détection identique pour l'ensemble des observations avec un appareil Biocom, et par calcul des plages de fluorescence révélées par un anticorps couplé à la fluorescéine et dirigé contre l'anticorps spécifique de l'HSP.

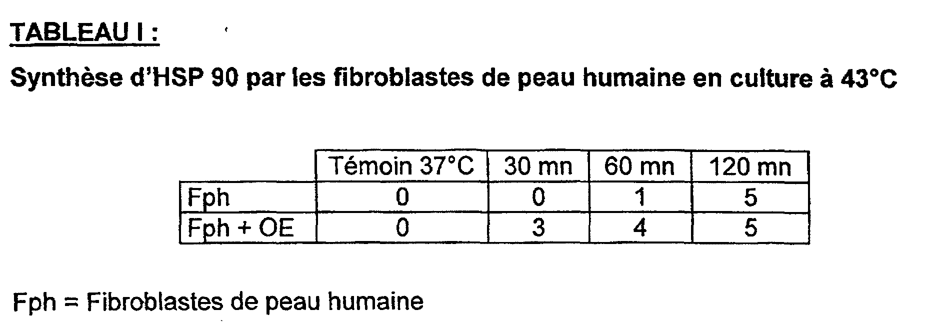

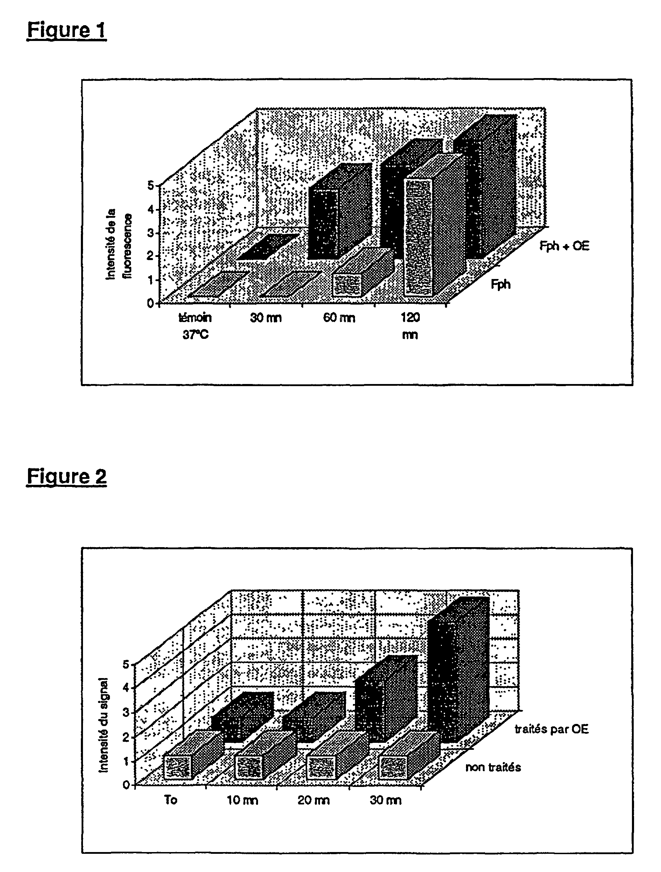

Le tableau 1 est illustré par la Figure 1.

L'extrait d'Opuntia, ajouté à la dose de 1 % dans le milieu de culture, n'exprime aucune activité après incubation d'une nuit à 37°C, alors que les mêmes fibroblastes de peau humaine cultivés à une température de 43°C, en présence d'extrait d'Opuntia, amènent l'apparition plus rapide de HSP 90.
Ainsi, la présence d'extrait d'Opuntia (OE) dans le milieu de culture permet une activation du processus de synthèse de HSP 90 par rapport à ce que développe la cellule non traitée. La synthèse n'est pas amplifiée, elle est activée plus rapidement. Au lieu d'intervenir normalement après 120 mn, la synthèse de HSP 90, par les fibroblastes en présence d'OE, se déclenche à 30 minutes après l'élévation de la température à 43°C (cf. Figure 1).
Au cours d'une agression thermique, OE permet une meilleure préservation du matériel cellulaire grâce au déclenchement rapide du processus de synthèse des HSP.

Il a été possible de préciser au cours d'une autre expérimentation que l'apparition de HSP 90 par les fibroblastes de peau humaine, issus d'un explant chirurgical provenant d'un sujet âgé de 5 ans soumis à un stress thermique de 43 °C, se fait entre 10 et 20 minutes, alors que les cellules provenant du même enfant mais non traitées par l'extrait de Cactacées ne font apparaître HSP 90 qu'à partir de 60 min.

Les groupes de données figurant dans les 3 tableaux sont indépendants les uns des autres, et ne peuvent donc être comparés.

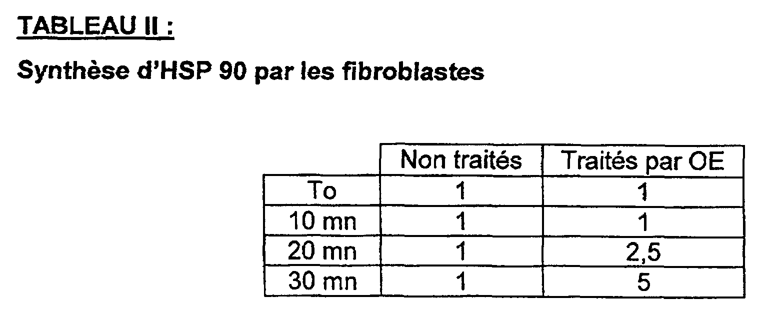

Le tableau II est illustré par la Figure 2.

### EXEMPLE II

### Synthèse des protéines de choc thermique (HSP) par les cellules épithéliales humaines.

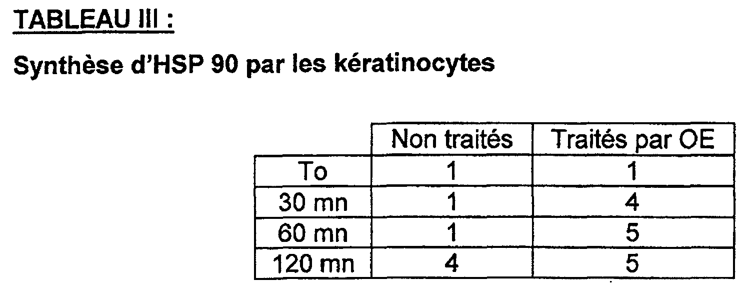

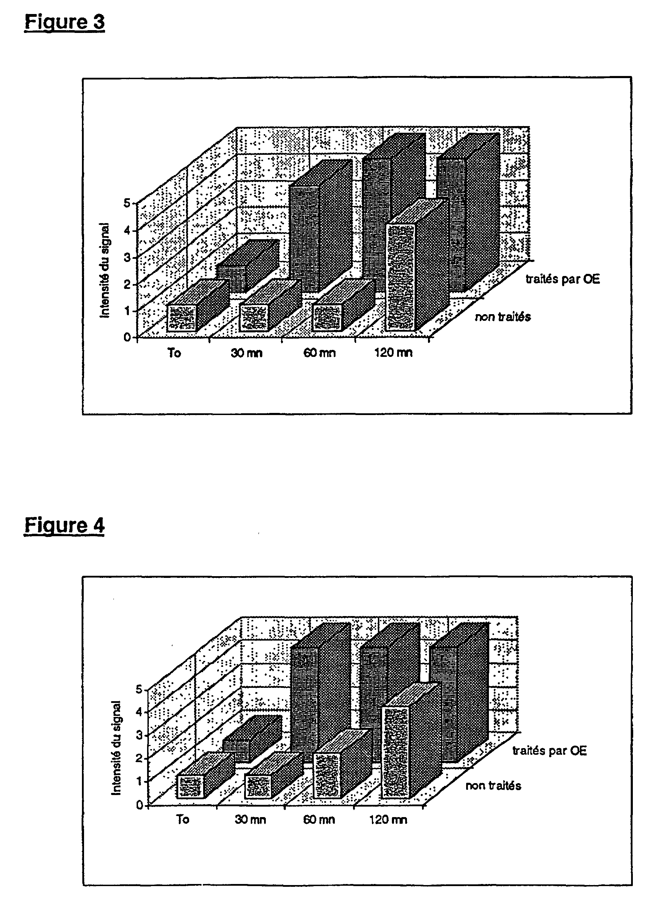

Le tableau III est illustré par la Figure 3.

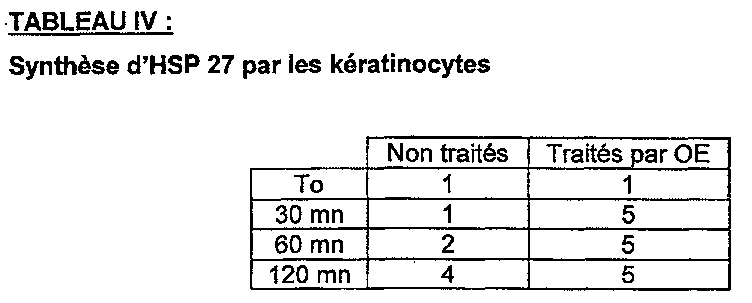

Le tableau IV est illustré par la Figure 4.

### EXEMPLE III

### Etude de la cytocompatibilité de l'extrait d'Opuntia

L'extrait de plantes ayant le métabolisme CAM doit être non cyto-toxique pour ne pas induire de synthèse d'HSP à cause de sa toxicité. Les études de cytocompatibilité ont été conduites sur les fibroblastes et les kératinocytes. Deux types d'essais ont été effectués : l'activité mitochondriale et la prolifération des cellules.
L'activité mitochondriale est évaluée par le test XTT.
Le test XTT est un test colorimétrique basé sur la réduction d'un sel de tétrazolium soluble (XTT = 2,3-bis [2-Methoxy-4-nitro-5-sulfophenyl]-2H-tetrazolium-5-carboxanilide), (si dérivé insoluble : test MTT = [3-(4,5-dimethyl thiazol-2-yl)-2,5-diphenyl-2H-tetrazolium bromide], par la NADPH réductase mitochondriale en un colorant du type formazan bleu violet.
Par exemple, 2.10⁴ cellules sont cultivées classiquement et traitées par diverses concentrations de l'actif à tester. La lecture se fait à 24 H ou à 48 H.
Le test XTT donné une valeur de l'activité mitochondriale, valeur donnée en densité optique (D.O.) à la longueur d'onde associée au bleu de formazan.
La prolifération des cellules est réalisée par numération à l'aide d'un compteur de cellules. Elle s'exprime en nombre de cellules.

### A) Test sur les fibroblastes :

Les fibroblastes proviennent d'explant de peau humaine prélevé sur un enfant âgé de 3 ans (cellules au troisième passage).

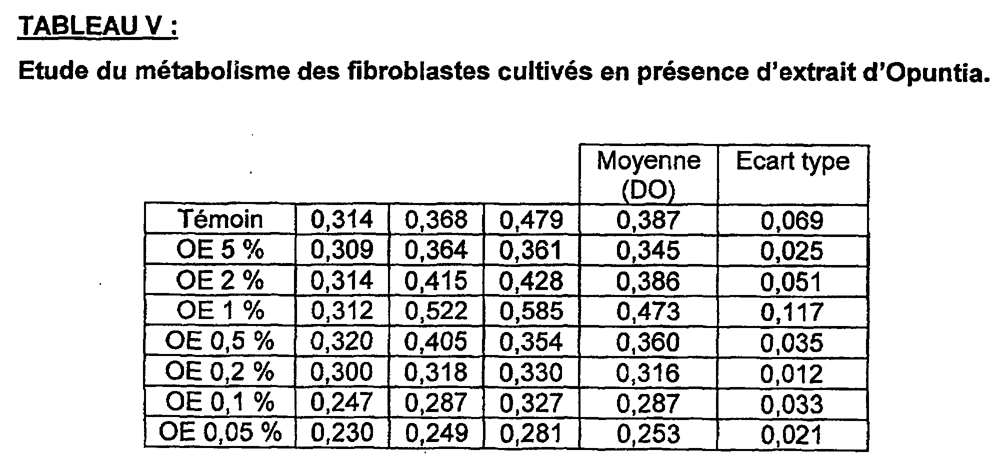

L'extrait d'Opuntia ne modifie pas le métabolisme des fibroblastes : les valeurs ne sont pas statistiquement significatives par rapport au témoin.

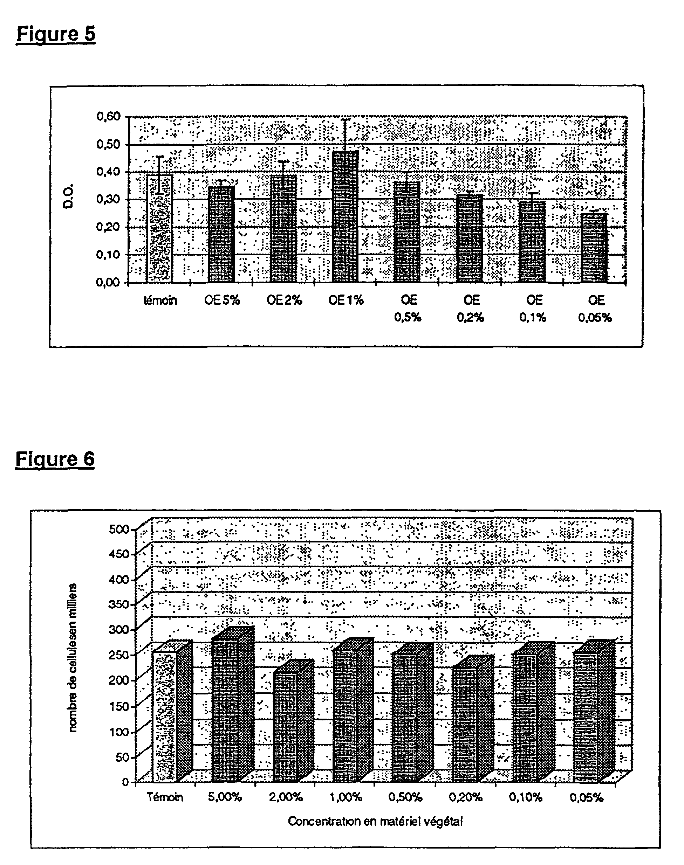

Le tableau V est illustré par la figure 5.

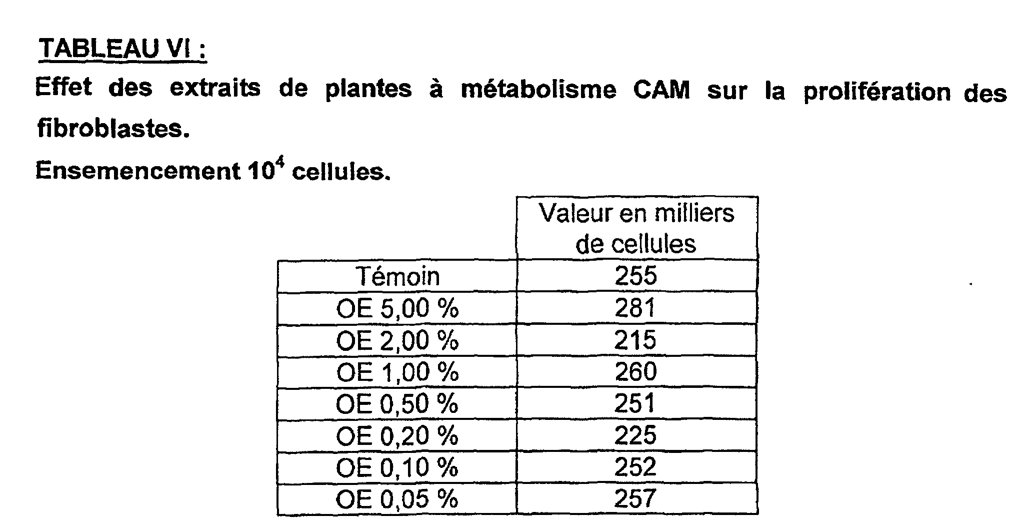

L'extrait d'Opuntia ne modifie pas la prolifération des fibroblastes après deux jours d'incubation.

Le tableau VI est illustré par la figure 6.

### B) Test sur les kératinocytes.

Les kératinocytes proviennent d'explant de peau humaine provenant de prépuces d'enfants âgés de 2 ans.

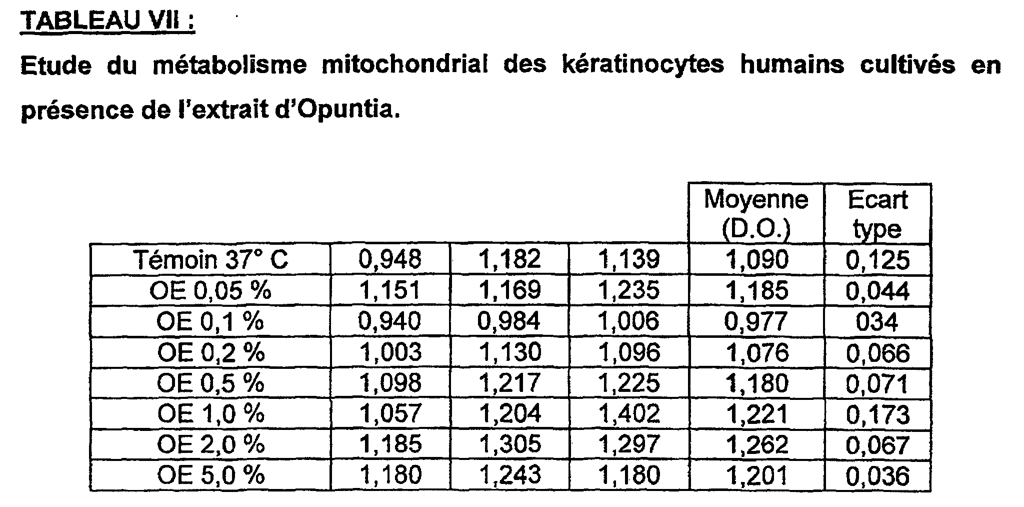

L'extrait d'Opuntia est cytocompatible avec les kératinocytes.
On n'observe pas de variation statistiquement significative de l'activité mitochondriale des kératinocytes cultivés avec des concentrations d'extrait d'Opuntia variant de 0,05 % à 5 %.

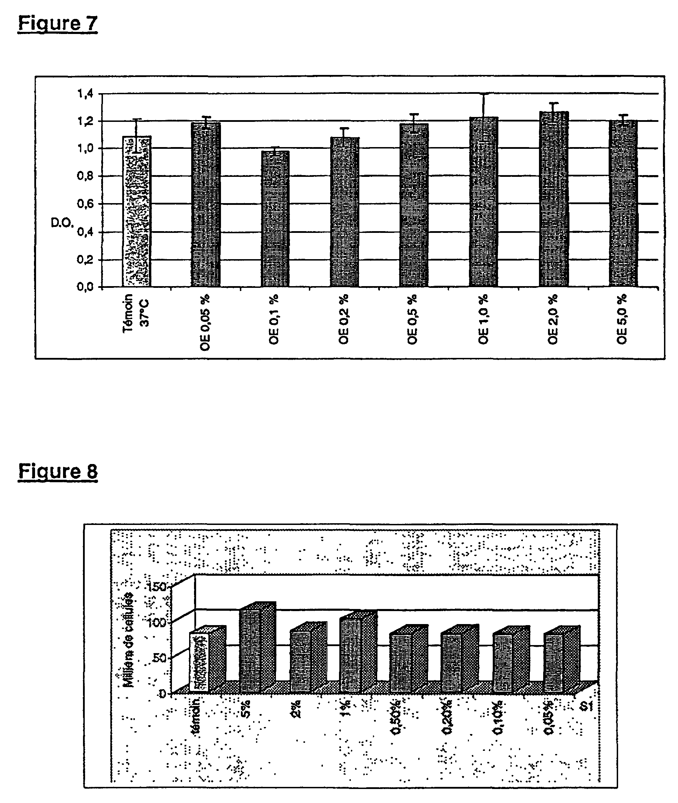

Le tableau VII est illustré par la figure 7.

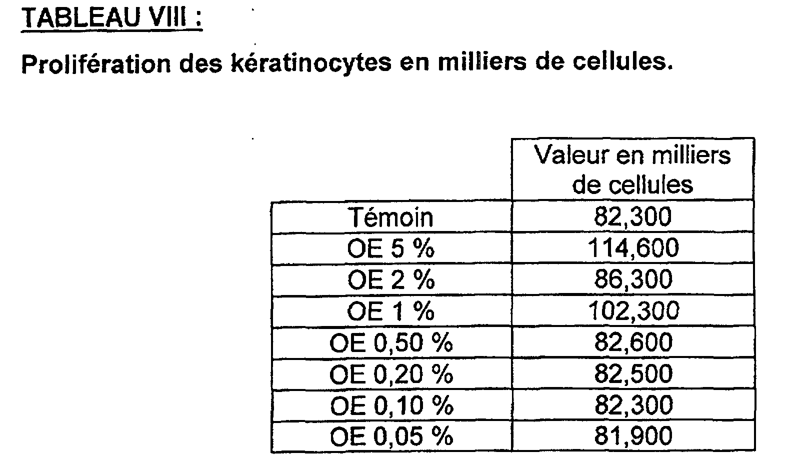

L'extrait d'Opuntia ne modifie pas la prolifération des kératinocytes.

Le tableau VIII est illustré par la figure 8.

### EXEMPLE IV

### Etude de l'influence de différentes doses d'extrait de plantes à métabolisme CAM sur la viabilité des kératinocytes soumis à un traitement thermique d'une heure à 48° C.

Après 30 minutes de contact avec l'extrait d'Opuntia, les kératinocytes sont incubés une heure à 48° C.
La viabilité des cellules est appréciée par test XTT.

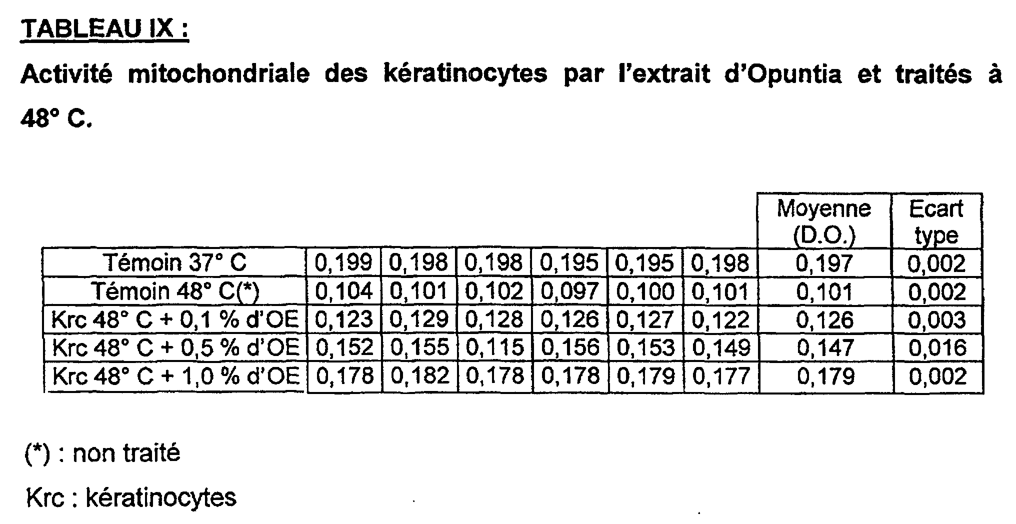

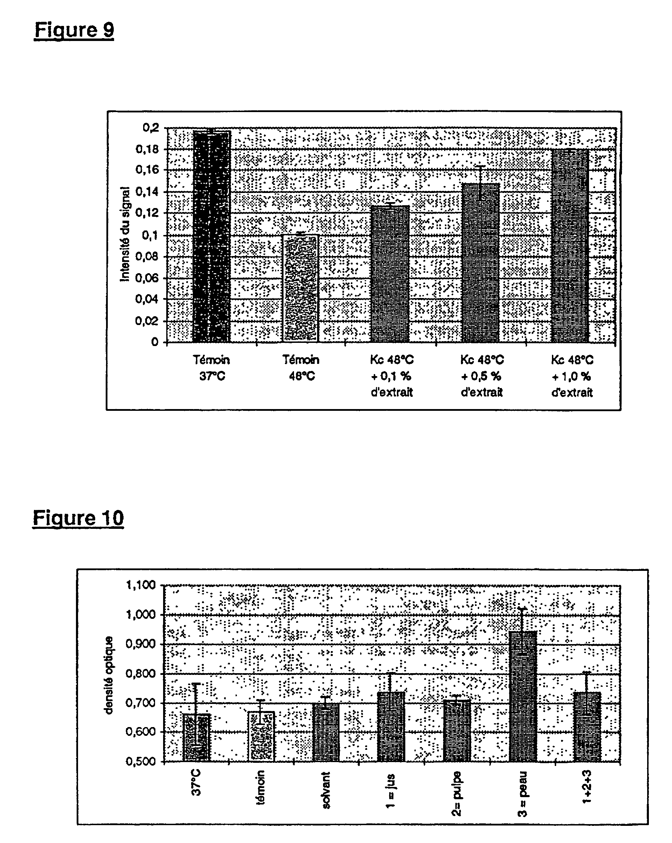

Le tableau IX est illustré par la figure 9.

L'effet thermoprotecteur est dose dépendant et apparaît avec l'utilisation de 10 µl par puits de culture de 1 ml obtenu par macération de 1 g de plantes dans 10 ml de solvant organique anhydre polaire.

### EXEMPLE V

### Effet thermoprotecteur des extraits de plante à métabolisme CAM.

### Etude de la viabilité des cellules traitées avec des extraits d'Opuntia obtenus avec les différentes parties de la plante.

Les conditions du test XTT sur les fibroblastes de peau humaine sont les suivantes
- Incubés une nuit à 37°C avec ou sans OE (témoin choc thermique)
- Premier choc thermique de 20 mn à 48° C
- Incubation de 40 mn à 37° C (production d'HSP)
- Deuxième choc pendant 20 mn à 50° C
- 15 mn à 37°C
- Test XTT pendant 3 heures à 37° C

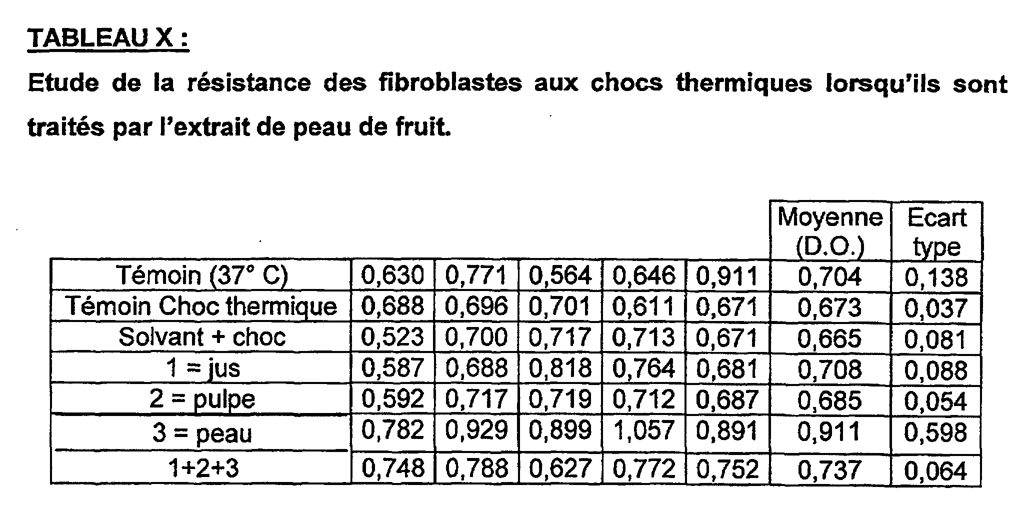

Le témoin à 37° C est cultivé sur une autre plaque.
Les densités optiques (D.O) mesurées lors du test XTT pour la peau des fruits sont statistiquement significatives par rapport à toutes les autres moyennes de densité optique sauf dans le cas de la solution totale (1+2+3).

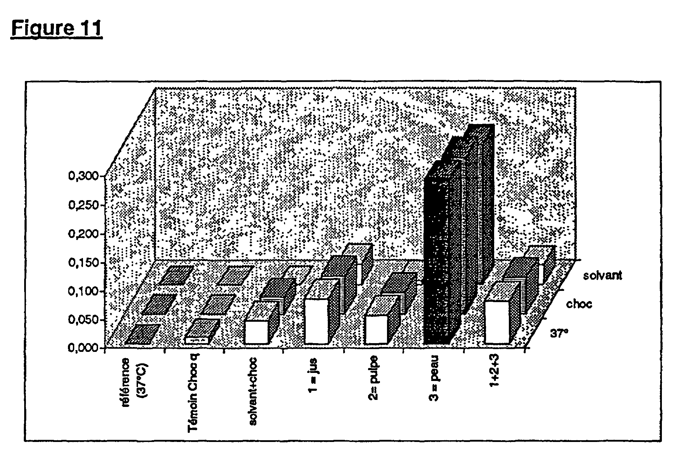

Ceci montre que pour certaines Cactacées la totalité de l'effet est contenue dans la peau.
Les fibroblastes résistent mieux aux chocs thermiques lorsqu'ils sont traités par l'extrait de la peau du fruit.
Le tableau X est illustré par la figure 10.

### EXEMPLE VI

### Etude de la viabilité des cellules traitées avec des extraits d'Opuntia obtenus avec les différentes parties de fruits.

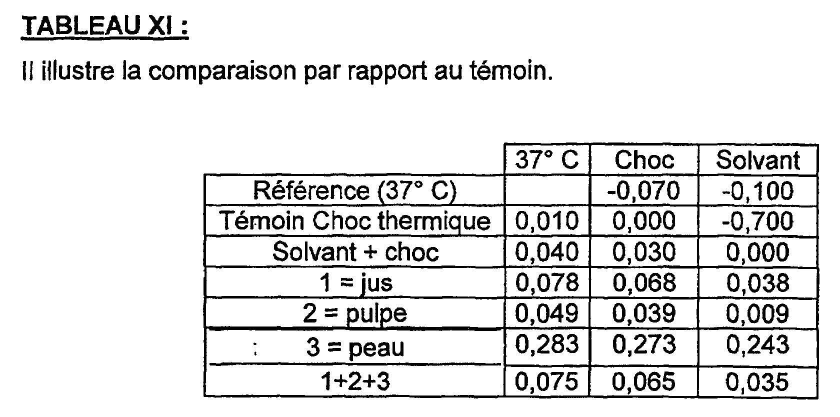

Les fibroblastes résistent mieux aux chocs thermiques lorsqu'ils sont traités par l'extrait de la peau du fruit.

Le tableau XI est illustré par la figure 11.

## Revendications

1. Substances à activité biologique, non cyto-toxiques, accélérant et/ou amplifiant la synthèse des protéines de choc thermique (HSP) isolées à partir de la peau du fruit des plantes à métabolisme CAM.

2. Substances à activité biologique selon la revendication 1, **caractérisées en ce que** le fruit des plantes à métabolisme CAM est un fruit d'Opuntia.

3. Substances à activité biologique selon l'une des revendications 1 ou 2, **caractérisées en ce qu'**elles sont obtenues à partir de la peau de fruits d'Opuntia et spécifiquement de la figue de Barbarie.

4. Procédé d'obtention de substances à activité biologique selon l'une des revendications 1 à 3, **caractérisé en ce que** l'on soumet un fruit d'une plante à métabolisme CAM, mature ou non mature, à un séchage, puis à une déshydratation, suivie d'un épuisement par un solvant organique polaire pour obtenir un extrait organique de fruit, dilue cet extrait avec de l'eau, purifie l'extrait dilué par épuisement à l'hexane, sépare la fraction organique hexanique que l'on évapore à sec sous vide, et obtient la fraction active du fruit de plante à métabolisme CAM.

5. Procédé d'obtention des substances à activité biologique selon la revendication 4, **caractérisé en ce que** l'on utilise en tant que solvant organique polaire, un solvant choisi parmi les alcools aliphatiques et les cétones aliphatiques.

6. Procédé d'obtention selon les revendications 4 et 5, **caractérisé en ce que** l'on utilise la peau de fruit de plante à métabolisme CAM, obtenue par pelage, érosion ou broyage du fruit.

7. Compositions pharmaceutiques, cosmétiques et/ou alimentaires, accélérant et amplifiant la synthèse des protéines de choc thermique (HSP), **caractérisées en ce qu'**elles renferment à titre de principe actif une ou plusieurs substances à activité biologique selon l'une des revendications 1 à 3, en association ou en mélange avec un excipient ou un véhicule inerte, non toxique, approprié à l'usage envisagé, et éventuellement un ou plusieurs principes actifs à action complémentaire.

8. Compositions pharmaceutiques, cosmétiques et/ou alimentaires selon la revendication 7, dans lesquelles l'excipient ou le véhicule inerte est l'un de ceux qui conviennent pour l'administration par voie digestive, parentérale, topique ou rectale.

9. Compositions pharmaceutiques, cosmétiques et/ou alimentaires selon les revendications 7 et 8, dans lesquelles la teneur en principe actif s'échelonne de 10 x 10⁻³ mg/ml à 400 mg/ml par prise unitaire.

10. Utilisation de la ou des substances à activité biologique selon l'une des revendications 1 à 3, pour accélérer et amplifier la synthèse des protéines de choc thermique (HSP) dans des milieux de culture cellulaire.

## Patentansprüche

1. Biologisch aktive Substanzen, die nicht zytotoxisch sind, die Synthese der Hitzeschockproteine (HSP) beschleunigen und/oder verstärken und von der Schale der Furcht der Pflanzen mit CAM-Stoffwechsel isoliert werden.

2. Biologisch aktive Substanzen nach Anspruch 1, **dadurch gekennzeichnet, dass** die Frucht der Pflanzen mit CAM-Stoffwechsel eine Opuntiafrucht ist.

3. Biologisch aktive Substanzen nach einem der Ansprüche 1 oder 2, **dadurch gekennzeichnet, dass** sie aus der Schale der Opuntiafrüchte und spezifischer der Kaktusfeige gewonnen werden.

4. Verfahren zur Herstellung von biologisch aktiven Substanzen nach einem der Ansprüche 1 bis 3, **dadurch gekennzeichnet, dass** die reife oder nicht reife Frucht einer Pflanze mit CAM-Stoffwechsel einer Trocknung und sodann einer Dehydratisierung unterzogen wird, gefolgt von einem Ausziehen durch ein polares organisches Lösemittel, um einen organischen Fruchtextrakt zu erhalten, dieser Extrakt mit Wasser verdünnt wird, der verdünnte Extrakt durch Ausziehen mit Hexan gereinigt wird, die organische Hexan-Fraktion abgeschieden wird, die im Vakuum bis zur völligen Trockenheit eingedampft wird, und die aktive Fraktion der Frucht der Pflanze mit CAM-Stoffwechsel gewonnen wird.

5. Verfahren zur Herstellung von biologisch aktiven Substanzen nach Anspruch 4, **dadurch gekennzeichnet, dass** als polares organisches Lösemittel ein Lösemittel verwendet wird, das aus den aliphatischen Alkoholen und den aliphatischen Ketonen gewählt wird.

6. Herstellungsverfahren nach den Ansprüchen 4 und 5, **dadurch gekennzeichnet, dass** die Schale der Frucht der Pflanze mit CAM-Stoffwechsel verwendet wird, die durch Schälen, Abtragen oder Zerstoßen der Frucht gewonnen wird.

7. Pharmazeutische, kosmetische und/oder Nahrungsmittel-Zusammensetzungen, die die Synthese der Hitzeschockproteine (HSP) beschleunigen und verstärken, **dadurch gekennzeichnet, dass** sie als Wirkstoff eine oder mehrere biologisch aktive Substanzen nach einem der Ansprüche 1 bis 3, in Verbindung oder gemischt mit einem inaktiven, nicht toxischen Hilfsstoff oder Träger, der für die beabsichtigte Verwendung geeignet ist, und eventuell einen oder mehrere Wirkstoffe mit komplementärer Wirkung enthält.

8. Pharmazeutische, kosmetische und/oder Nahrungsmittel-Zusammensetzungen nach Anspruch 7, wobei der inaktive Hilfsstoff oder Träger einer von jenen ist, die für die Verabreichung auf oralem, parenteralem, topischem oder rektalem Weg geeignet sind.

9. Pharmazeutische, kosmetische und/oder Nahrungsmittel-Zusammensetzungen nach den Ansprüchen 7 und 8, wobei der Gehalt an aktivem Wirkstoff zwischen 10 x 10⁻³ mg/ml und 400 mg/ml pro Einnahmeeinheit gestaffelt ist.

10. Verwendung der biologisch aktiven Substanz oder Substanzen nach einem der Ansprüche 1 bis 3, um die Synthese der Hitzeschockproteine (HSP) in Zellkulturmilieus zu beschleunigen und zu verstärken.

## Claims

1. Substances with biological activity, non cytotoxic, which speed up and/or amplify the synthesis of heat shock proteins (HSP) isolated from the skin of the fruits of the plants with CAM metabolism.

2. Substances with biological activity according to claim 1, **characterized in that** the fruit of the plants with CAM metabolism is a fruit of Opuntia.

3. Substances with biological activity, according to one of the claims 1 or 2, **characterized in that** they are obtained from the skin of the fruits of Opuntia and specifically from that of Prickly Pear.

4. A process for obtaining the substances with biological activity according to one of the claims 1 to 3, **characterized in that** a fruit of a plant with CAM metabolism, mature or non mature, is submitted to drying, then to deshydratation followed by an extraction with an organic polar solvent to obtain an organic extract of the fruit, then dilutes this extract by extracting it with hexane, then separates the organic hexanic moiety which is evaporated to dryness under vaccum and one obtains the active portion of the fruit of a plant with CAM metabolism.

5. A process for obtaining the substances with biological activity according to claim 4, **characterized in that** as a polar organic solvent, a solvent selected from among aliphatic alkanols and aliphatic ketones is used.

6. A process of obtention according to claims 4 and 5, **characterized in that** the skin of the fruit of a plant with CAM metabolism obtained by peeling off, erosion or grinding of the fruit, is used.

7. Pharmaceutical, cosmetic and/or alimentary compositions which speed up and amplify the synthesis of heat shock proteins (HSP) **characterized in that** they contain as an active ingredient one or several substances with biological activity according to one of the claims 1 to 3, in combination or admixture with an inert, non toxic, carrier suitable for the intended use and optionally one or several active ingredients having a complementary action.

8. Pharmaceutical, cosmetic and/or alimentary composition according to claim 7, wherein the inert carrier or vehicle is one of those suitable for the administration by digestive, parenteral, topic or rectal way.

9. Pharmaceutical , cosmetic and/or alimentary compositions according to claims 7 and 8 wherein the concentration of the active ingredient ranges form 10 x 10⁻³ mg/ml to 400 mg/ml per unit dosage.

10. Use of the substance or the substances with biological activity according to one of the claims 1 to 3, to speed up and amplify the synthesis of heat shock proteins (HSP) in the cell culture mediums.
